Europäisches Patentamt

(19) European Patent Office

Office européen des brevets

(11) Publication number : **0 461 869 A2**

# (12) EUROPEAN PATENT APPLICATION

(21) Application number : 91305283.3

(22) Date of filing : 12.06.91

(51) Int. Cl.$^5$: **A61K 37/02, C07K 5/10,** C07K 15/00

(30) Priority : 12.06.90 US 536840
17.05.91 US 700232

(43) Date of publication of application :
18.12.91 Bulletin 91/51

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Gibbs, Jackson B.
118 Tartan Terrace
Chalfont, PA 18914 (US)
Inventor : Dixon, Richard A.F.
758 Hartley Drive
Lansdale, PA 19446 (US)
Inventor : Garsky, Victor M.
752 Palmer Place
Blue Bell, PA 19422 (US)
Inventor : Schraber, Michael D.
839 Price Road
Harleysville, PA 19438 (US)

(74) Representative : Thompson, John Dr. et al
Merck & Co., Inc. European Patent
Department Terlings Park Eastwick Road
Harlow, Essex CM20 2QR (GB)

(54) Chemotherapeutic agents.

(57) Compounds which inhibit farnesylation of Ras protein, chemotherapeutic compositions comprising one or more compounds of the invention, and methods for inhibiting farnesylation of Ras protein.

EP 0 461 869 A2

## BACKGROUND OF THE INVENTION

The ras oncogene protein, Ras, must be localized in the plasma membrane in order to function and transform normal cells into cancer cells. (Gibbs et al. Microbiol. Rev. 53:171-286 (1989)) Localization occurs upon Ras protein farnesylation. Forms of Ras in cancer cells have mutations that distinguish the protein from Ras in normal cells. At least 3 post-translational modifications are required for this localization, and all 3 modifications occur at the C-terminus of Ras. The Ras C-terminus contains a sequence motif termed a "Cys-Aaa[1]-Aaa[2]-Xaa" box (Aaa is an aliphatic amino acid, and Xaa is any amino acid) (Willumsen et al. Nature 310:583-586 (1984)). Other proteins having this motif include the ras-related GTP-binding proteins such as Rho, fungal mating factors, the nuclear lamins, and the gamma subunit of transducin..

Farnesylation of Ras by the isoprenoid farnesyl occurs in vivo on Cys via a thio-ether linkage (Hancock et al., Cell 57:1167 (1989); Casey et al., Proc. Natl. Acad. Sci. USA 86:8323 (1989)). In addition, Ha-Ras and N-Ras are palmitoylated via a thioester on a Cys residue near the C-terminal Cys farnesyl acceptor (Gutierrez et al. EMBO J. 8: 1093-1098(1989); Hancock et al., Cell 57: 1167-1177(1989)). Ki-Ras lacks the palmitate acceptor Cys. The last 3 amino acids at the Ras C-terminal end are removed proteolytically, and methyl esterification occurs at the new C-terminus (Hancock et al. ibid). Fungal mating factor and mammalian nuclear laminins undergo identical modification steps (Anderegg et al., J. Biol. Chem. 263:18236 (1988); Farnsworth et al. J. Biol. Chem. 264:20422 (1989)).

Inhibition of Ras farnesylation in vivo has been demonstrated with lovastatin (Merck & Co., Rahway, NJ) and compactin (Hancock et al. ibid; Casey et al. ibid; Schafer et al. Science 245:379 (1989). These drugs inhibit HMG-CoA reductase, the rate-limiting enzyme for the production of polyisoprenoids, the farnesyl precursor. It is speculated that a putative farnesyl-protein transferase using farnesyl-PPi(FPP)[1] as a precursor is responsible for Ras farnesylation (Goldstein et al. Nature 343:425-430(1990)).

## SUMMARY OF THE INVENTION

The invention includes compounds which inhibit the enzyme-catalyzed farnesylation of ras oncogene protein, chemotherapeutic compositions comprising one or more compounds of the invention, and methods for inhibiting farnesylation of Ras protein via inhibition of the farnesyl-protein transferase enzyme.

Compounds of the invention comprise the amino acid sequence

$$\text{Cys - Aaa}^1 \text{ - Aaa}^2 \text{ - Xaa}$$

wherein

Aaa[1] is an aliphatic amino acid;

Aaa[2] is an aliphatic amino acid; and

Xaa is any amino acid.

Aliphatic amino acids include Ala, Val, Leu and Ile.

Preferred compounds are those where Aaa[1] is Val. Preferred compounds are also those where Aaa[2] is Leu, Ile or Val. Preferred compounds are also those where Xaa is Ser or Met. More preferred compounds are

Cys-Val-Leu-Ser;

Cys-Val-Ile-Met; and

Cys-Val-Val-Met.

Inhibition of Ras protein farnesylation blocks the ability of Ras to transform normal cells to cancer cells. The compounds of the invention inhibit Ras localization in the plasma membrane and generate soluble Ras which can act as a dominant negative inhibitor of Ras function. While soluble Ras in cancer cells can become a dominant negative inhibitor, soluble Ras in normal cells would not be an inhibitor.

A cytosol-localized (no Cys-Aaa[1]-Aaa[2]-Xaa box membrane domain) and activated (impaired GTPase, staying bound to GTP) form of Ras acts as a dominant negative Ras inhibitor of membrane-bound Ras function (Gibbs et al. Proc. Natl. Acad. Sci. USA 86:6630-6634(1989)).

Cytosol-localized forms of Ras with normal GTPase do not act a inhibitors. Gibbs et al. ibid show this effect in Xenopus oocytes and in mammalian cells.

Administration of compounds of the invention to block Ras farnesylation not only decreases the amount of Ras in the membrane but also generates a cytosolic pool of Ras. In tumor cells having activated Ras, the cytosolic pool acts as another antagonist of membrane-bound Ras function. In normal cells having normal Ras, the cytosolic pool of Ras does not act as an antagonist. In the absence of complete inhibition of farnesylation, other farnesylated proteins are able to continue with their functions.

Farnesyl-transferase activity may be reduced or completely inhibited by adjusting the compound dose. Reduction of farnesyl-transferase enzyme activity by adjusting the compound dose would be useful for avoiding possible undersirable side effects such as interference with other metabolic processes which utilize the enzyme.

These compounds and their analogs are inhibitors of farnesyl-protein transferase. Farnesyl-protein transferase utilizes farnesyl-pyrophosphate to covalently modify Cys of the Ras box with a farnesyl group. Inhibition of farnesyl pyrophosphate biosynthesis by inhibition of HMG-CoA reductase blocks Ras membrane localization in vivo and inhibits Ras function. Inhibition of farnesyl-protein transferase is more specific and has fewer side effects than a general inhibitor or isoprene biosynthesis. Cys-Aaa$^1$-Aaa$^2$-Xaa box variations are identified below by single letter amino acid abbreviations.

## DETAILED DESCRIPTION OF THE INVENTION

Polyisoprenylation of Ras⁻ [$^3$H]mevalonic acid (35 Ci/mmol) and [$^3$H]farnesyl-PPi(FPP) FPP (20 Ci/mmol) were purchased from New England Nuclear. [$^{14}$C]FPP (49.7 mCi/mmol) was prepared enzymatically from partially purified prenyl transferase, [4-$^{14}$C]isopentyl-PPi (47.9 mCi/mmol, Amersham), and geranyl-PPi. Reactions contained 280 pmol (10 μCi) of [$^3$H]mevalonic acid, 500 pmol (0.025 μCi) of [$^{14}$C]FPP or 25 pmol (0.5 μci) of [$^3$H]FPP. In a standard 50 μl reaction, 250 pmol of Ras purified from E. coli (Gibbs et al. Proc. Natl. Acad. Sci. USA 86:6630-6634(1989)) was added to 35 μl of rabbit reticulocyte lysate (Promega, L4210) or a tissue extract (100,00 x g supernatant, 10 mg/ml). Buffer conditions were adjusted by the addition of one-tenth volume of 500 mM Tris-Cl, pH 8.0/50 mM MgCl$_2$ or one-tenth volume of 500 mM NaHepes, pH 7.5/50 mM MgCl$_2$/50mM dithiothreitol. In the Tris-buffered assay, the reactions were incubated for 1 hour at 24°C. Reaction velocities in the Hepes buffered assays were at least four times greater, and incubations were for 10 minutes at 24°C. Tissue extracts were prepared as described previously [(ethylenebis(oxyethylenenitrilo)]tetra-acetic acid in buffer containing 50 mM Tris-Cl, pH 8.0/1 mM EGTA/1 mM MgCl$_2$/1 mM dithiothreitol/10 μl/ml aprotinin/0.5 mM phenylmethlysulfonyl fluoride.

Ras proteins used as substrates were expressed in E. coli and purified. Gibbs et al. ibid, Gibbs et al. Proc. Natl. Acad. Sci. USA 85:5026-5030(1988). Ras proteins having different Cys-Aaa$^1$-Aaa$^2$-Xaa box sequences were constructed with oligonucleotide linkers as described in Gibbs et al. ibid. E. coli-expressed Rho was a gift of J.C. Lacal. Geraniol and trans. trans farnesol were purchased from Aldrich. FPP, geranyl-PPi, and dimethylallyl-PPi were synthesized as described in Davisson et al. Methods Enzymol 110:130-144(1985). Geranylgeraniol and geranylgeranyl-PPi were obtained from Dr. Tetsuo Takigawa (Kuraray Co., LTD., Tokyo) and Kyozo Ogura (Tohoku Univ., Sendai), respectively.

The compounds can be synthesized from their constituent amino acids by conventional peptide synthesis techniques, preferably by solid-phase technology. The peptides are then purified by reverse-phase high performance liquid chromatography (HPLC).

Standard methods of peptide synthesis are disclosed, for example, in the following works: Schroeder et al., "The Peptides", Vol. I, Academic Press 1965, or Bodanszky et al., "Peptide Synthesis", Interscience Publishers, 1966, or McOmie (ed.) "Protective Groups in Organic Chemistry", Plenum Press, 1973, or Barany et al., "The Peptides: Analysis, Synthesis, Biology" 2, Chapter 1, Academic Press, 1980. The teachings of these works are hereby incorporated by reference.

Peptides were prepared with an Applied Biosystems model 430A peptide synthesizer, purified by reverse-phase HPLC, and characterized by amino acid analysis and fast atom bombardment mass spectrometry. Chemical farnesylation was achieved by reacting trans, trans-farnesyl bromide (Aldrich) with unprotected peptide.

Enzyme reactions were analyzed by SDS-PAGE (sodium dodecyl sulfate/polyacrylamide gel electrophoresis) or immunoprecipitation. For gel analysis, each labeling reaction was boiled for 3 min in buffer containing 1% SDS/100 mM Tris-Cl, pH 6.8/10% glycerol/1% 2-mercaptoethanol, and loaded directly on 13.7% polyacrylamide gels. Radiolabeled Ras was visualized by autoradiography after fluorographic enhancement with Enlightning (New England Nuclear). For immunoprecipitation of radiolabelled Ras, reactions were first extracted with Triton X-114 using a modification of the procedure of Bordier (J. Biol. Chem. 256:1604-1607(1981). An aliquot (150 μl) of 1% Triton X-114 (Pierce) was added to the 50 μl reaction mix to achieve a final Triton X-114 concentration of 0.75%. Following 10 min. on ice, the tubes were transfered to a 37°C bath for 3 min. The cloudy solution was then centrifuged at 10,000 x g for 5 min. at room temperature. The upper aqueous phase was removed, and the detergent phase was diluted by the addition of 150 μl cold phosphate-buffed saline plus 150 μl of Ras monoclonal antibody Y13-259 coupled to Sepharose-protein A beads (Gutierrez et al. EMBO J. 8:1093-1098(1989) and Hancock et al. Cell 57:1167-1177(1989).

Assays could also be analyzed by another method which was more rapid. After thermally pre-equilibrating the assay mixture in the absence of enzyme, reactions were initiated by the addition of isoprenyl-protein transferase and stopped at timed intervals by the addition of 1M HCl in ethanol (1 mL). The quenched reactions were allowed to stand for 15 m (to complete the precipitation process). After adding 2 mL of 100% ethanol, the reactions were vacuum-filtered through Whatman GF/C filters. Filters were washed four times with 2 mL aliquots of 100% ethanol, mixed with scintillation fluid (10 mL) and then counted in a Beckman LS3801 scintillation counter.

Chemical Cleavage and Identification of the Isoprenoid Moiety on Ras- Following Triton X-114 extraction and immunoprecipitation of radiolabeled Ras, the protein was chemically treated by a modification of procedures described in Casey et al. Proc. Natl. Scad. Sci. USA 86:8323-8327 (1989); Maltese et al. J. Biol. Chem. 264:18168-18172 (1989). All treatments were at 4° C unless otherwise stated. The immune complex was precipitated by the addition of 10 volumes of 15% trichloroacetic acid, and then delipidated by 3 sequential treatments (minimum 6 hr each) with 1 ml acetone. The acetone pellets were then incubated twice for 16 hours with 750 µl $CHCl_3$/Methanol (1:2). The pellets were then dried under a gentle stream of nitrogen and resuspended in 600 µl 0.5% formic acid. Chemical cleavage was initiated by the addition of 100 µl $CH_3I$ (Aldrich). Reactions were incubated for 16 hr at 37° C in the dark. An aliquot (30 µl) of 35% $Na_2CO_3$ was added, followed by an overnight room temperature incubation. Reactions were extracted 3 times with 400 µl each of $CHCl_3$/Methanol (9:1). The lower organic phases were pooled, evaporated under nitrogen, resuspended in 200 µl solvent A (50% $CH_3CN$/25 mM H $P_3O_4$) and analyzed by HPLC. An aliquot of the extracted cleavage product was applied to a 0.5 x 15 cm $C_{18}$ HPLC column (Vydac). Elution was achieved at 1 ml/min using a 22.5 ml linear gradient of 0-15% solvent B (100% $CH_3CN$/25 mM $H_3PO_4$), followed by a steeper gradient (56.5 ml at 1 ml/min) of 15-100% solvent B. The absorption profile of the elution was monitored by measuring absorbance at 210 nm. Fractions (25 µl) were collected and measured for radioactivity in 2.5 ml Radi-Safe scintillation fluid (Beckman).

Farnesylation of Ras in vitro- To evaluate in vitro polyisoprenylation of Ras, we have used two engineered S.cerevisiae RAS gene products, [Leu[68]]RASI(term.) and [Leu[68]]RASI(term.)CVLS (CVLS=Cys-Val-Leu-Ser) Gibbs et al. Proc. Natl. Acad. Sci. USA86:6630-6634. Both proteins contain the first 184 amino acids of yeast RASI which do not have any Cys residues. [Leu[68]]RASI(term.) ends with a Pro substitution for Leu-185. [Leu[68]]RASI (term.)CVLS has the final Pro residue replaced by an additional seven C-terminal residues having the sequence -Ser-Leu-Lys-Cys-Val-Leu-Ser. Purified Ras proteins were incubated with rabbit reticulocyte lysate in the presence of 5 µM [$^3$H]mevalonic acid or 10 µM [$^{14}$C]FPP. Both radioactive precursors were incorporated into [Leu[68]]RASI(term.)CVLS. No detectable labeling was observed in reactions lacking Ras or having [Leu[68]] RASI(term.) suggesting that modification was occurring at the unique C-terminal region of [Leu[68]]RASI(term.)CVLS. Ras was positively identified as the acceptor protein by immunoprecipitation with the Ras-specific monoclonal antibody Y13-259. Both normal and oncogenic mammalian Ha Ras proteins also served as substrates. In addition, Aplysia Rho, which has the C-terminal sequence -Cys-Val-Val-Leu, was radiolabeled by [$^3$H]mevalonate.

Ras labeled by [$^3$H]mevalonic acid or [$^{14}$C]FPP was quantitatively extracted into a Triton X-114 detergent phase as has been described previously for Ras modified in vivo (Gutierrez et al. ibid and Hancock et al. ibid); Ras lacking incorporated radiolabel remained in the aqueous phase. This result indicated that the in vitro modification conferred hydrophobic properties to Ras.

The activity or activities responsible for radiolabel incorporation into Ras remained in the reticulocyte cytosol fraction upon centrifugation at 100,000 x g. To test whether **in vitro** polyisoprenylation of Ras could be detected in extracts of other tissues, 100,000 x g supernatant fractions were prepared from rat brain, heart, kidney, liver, lung, skeletal muscle, spleen, and testis. [$^3$H]FPP was incorporated into Ras during incubation with each supernatant tested; the highest incorporation was observed using the supernatant prepared from brain. Inclusion of 10 mM NaF in the assays as a phosphatase inhibitor did not influence the tissue distribution results. The high activity in brain may parallel the abundance of 20 kDa G-proteins in this tissue. Incorporation of [$^3$H]FPP into Ras and Rho was also detected using a bovine brain cytosolic fraction.

Cys-Aaa[1]-Aaa[2]-Xaa box mutations of [Leu [68]RASI(term.)CVLS PD wild type and ras-related proteins Rho and YPTI were tested as farnesylation substrates using [$^3$H]FPP and bovine brain cytosol as a source of farnesyl-protein transferase activity. The results, showing activity relative to the wild type, are shown in Table 1. The criticality of Cys-Aaa[1]-Aaa[2]-Xaa as defined in this specification is shown.

## Table 1

| Mutation (5μM) | | Farnesylation substrate |
|---|---|---|
| — | | |
| PD (wild type) | -KCVLS | +++ |
| Ser (188) | -KSVLS | − |
| Trp (191) | -KCVLW | + |
| Ala (192) | -KCVLSA | − |
| Term (188) | -K | − |
| Term (189) | -KC | − |
| Term (190) | -KCV | − |
| Term (191) | -KCVL | − |
| | | |
| Rho | -GCVVL | +/− |
| YPT1 | -GGGCC | − |

Relative values of activity:

|     |        |
|-----|--------|
| +++ | 1      |
| +   | 0.3    |
| −   | ≪ 0.01 |

No incorporation was observed with forms of Ras having a deletion of the Cys-Aaa[1]-Aaa[2]-Xaa box or a Cys to Ser substitution {[Leu[68]]RASI(term.)SVLS.

Using bovine brain cytosol, [3H]mevalonic acid was not incorporated into Ras unless the tissue extract was supplemented with a creatine phosphate/creatine kinase mixture similar to that found in the rabbit reticulocyte lysate preparation.

Two ras-related 20 kDa G-proteins were also tested. YPTI (C-terminal sequence -Gly-Gly-Cys-Cys) was not radiolabelled whereas Rho (-Cys-Val-Val-Leu) was a weak substrate. Ras proteins having an extended box (-Cys-Val-Leu-Ser-Ala) or a shortened sequence (-Cys-Val-Leu) were poor substrates; at 5μM, no radiolabel incorporation was detected. At 100 μM, 40- and 200-fold less radiolabel incorporation was observed for [Leu[68]]RASI (term.)CVLSA and [Leu[68]]RASI(term.)CVL, respectively, compared to the control [Leu[68]]RASI(term.)CVLS.

Ras protein radiolabeled by [3H]FPP and bovine brain cytosol was immunoprecipitated and then treated with iodomethane as described above in order to identify the product incorporated into Ras. This method cleaves thioether linkages by a sulfonium salt intermediate and produces the free alcohol form of the released isoprenoid. Recovery of radioisotope following treatment with iodomethane was 65%. Recovery of [3H]FPP standard in a parallel reaction lacking Ras was 61% indicating that the 35-40% loss occurred during the extraction procedures. The reaction products were analysed by HPLC using a C-18 column. A major peak representing 86% of the recovered radioactivity co-migrated with the farnesol standard. A similar profile was observed for Ras labeled with [3H]mevalonate in rabbit reticulocyte lysates. No peaks were detectable for assays having [Leu[68]]RASI(term.) which lacks the Cys acceptor site. Treatment of [3H]FPP-labeled [Leu[68]]RASI(term.)CVLS with 0.1M KOH/100% methanol did not release radioactivity. The observation that FPP served as presursor for polyisoprenylation of Ras and the identification of farnesol as the major iodomethane cleavage product indicates that a farnesyl-protein transferase activity is present in the cytosolic fraction of rabbit reticulocytes, bovine brain, and various rat tissues.

Properties of the bovine brain farnesylprotein transferase activity- Farnesylation of Ras was inhibited by EDTA; 5 mM $MgCl_2$ served as an effective source of divalent cation. By varying the [$^{14}$C]FPP concentration from 0.03-30 μM and the [Leu$^{61}$]Ha Ras concentration from 0.03-250 μM, we estimate apparent Km values of 0.3 μM and 1.0 μM for FPP and Ras, respectively. Using Ras complexed with either GTP[γ-S] or GDP[β-S] as substrate, equivalent farnesylation was observed suggesting that farnesylation was independent of guanine nucleotide-induced Ras structural conformation (Milburn et al. Science 247:939-945(1990)). The farnesyl transferase activity eluted from a Superose-12 gel filtration column as a single peak having an apparent size of 190 kDa. A smaller activity peak having a size of 75 kDa was observed when the protease inhibitors leupeptin and antipain were not included in the chromatography buffer.

To determine specificty of the farnesyl transferase activity, several isoprenoids and peptides were tested as competitors of the farnesylation reaction (Table 2). In assays having 0.5 μM [$^{3}$H]FPP and 1 μM Ras, nonradioactive FPP and geranylgeranyl-PPi potently inhibited the assay Ras is not significantly modified by geranylgeranyl in vivo (Casey et al. Proc. Natl. Acad. Sci. USA (1989) 86:8323-8327) or in vitro when [$^{3}$H]mevalonate is used as a precursor of polyisoprene biosynthesis. Geranyl-PPi and dimethylallyl-PPi were less potent as competitors whereas farnesol and geranylgeraniol were inactive.

Several peptides having the Harvey Ras Cys-Aaa$^{1}$-Aaa$^{2}$-Xaa box sequence were also tested (Table 2). The smallest peptide, CVLS (Cys-Val-Leu-Ser), was nearly as potent as the larger peptides indicating that the critical interactions between Ras and the farnesyl-protein transferase are confined to the 4 residue box region. The sulfhydryl moiety is apparently essential for competition because peptides having a Cys to Ser substitution were inactive. Peptides lacking the Cys-Aaa$^{1}$-Aaa$^{2}$-Xaa box sequence, free Cys, S-(trans, trans)farnesyl-Cys, and unrelated peptides having a single Cys residue were not competitors when tested up to 100 μM. Initial experiments with peptide SSGCVLS indicate that it is a substrate for farnesylation. The predicted product of the smallest active peptide, S-(trans, trans) farnesyl-CVLS, was approximately 10-fold less potent than peptide alone (Table 2). CVIM and S-(trans, trans) farnesyl-CVIM were more potent than the respective CVLS derivatives.

Table 2. Competition of Ras farnesylation by polyisoprenoids and peptides.

| Compound | $IC_{50}$ (μM) |
|---|---|
| Dimethylallyl-PPi | 325 |
| Geranyl-PPi | 67 |
| Farnesyl-PPi | 0.7 |
| Geranylgeranyl-PPi | 1.4 |
| Farnesol | >>100 |
| Geranylgeraniol | >>100 |
| YRKLNPPDESGPGSMSSKCVLS | 1.4 |
| SSGCVLS | 2.0 |
| SSGSVLS | >>200 |
| CVLS | 2.2 |
| CVIM | 0.09 |
| SVLS | >>100 |
| S-(trans, trans) farnesyl-CVLS | 28 |
| S-(trans, trans) farnesyl-CVIM | 1.4 |

Farnesyl-protein transferase from bovine brain was chromatographed on DEAE-Sephacel (Pharmacia, 0-0.8 M NaCl gradient elution) and N-octyl agarose (Sigma, 0-0.6 M NaCl gradient elution).

[Leu$^{68}$]RASI(term.)CVLS at 1 μM, 0.5 μM [$^{3}$H]FPP, and the indicated compounds were incubated with this partially purified enzyme preparation for 10 min at 24° C using the Hepes-buffered assay described in above. Reactions were analyzed by immunoprecipitation without the Triton X-114 extraction step followed by Nitrocellulose filtration. Data are the average of 2-5 determinations. >>, No competition at the highest concentration tested. Single capital letter amino acid abbreviations are used above.

These compounds may be administered by any convenient means which will result in inhibition of Ras protein farnesylation and subsequent transformation of normal cells into cancer cells Such administration may be by continuous intravenous, bolus injection or oral administration.

Suitable compositions of the invention include aqueous solutions comprising compounds of the invention and pharmacologically acceptable carriers, e.g. saline, at a pH level of, for example, 7.4. The solutions may be introduced into a patient's intramuscular blood-stream by local bolus injection.

## Claims

1. A compound which inhibits plasma membrane Ras protein localization and prevents transformation of normal cells into cancer cells, which compound comprises the amino acid sequence.

   cys-Aaa$^1$-Aaa$^2$-Xaa

   wherein
   Aaa$^1$ is an aliphatic amino acid;
   Aaa$^2$ is an aliphatic amino acid; and
   Xaa is any amino acid.

2. A compound of Claim 1 wherein
   Aaa$^1$ is Ala, Val, Leu or Ile;
   Aaa$^2$ is Ala, Val, Leu or Ile, and
   Xaa is Ser or Met.

3. A compound of Claim 2 which is Cys-Val-Leu-Ser.

4. A compound of claim 2 which is cys-Val-Ile-Met.

5. A compound of Claim 2 which is cys-Val-Val-Met.

6. A chemotherapeutic composition comprising a compound of Claim 1.

7. The use of a compound as claimed in Claim 1 for the manufacture of a medicament for inhibiting farnesylation of Ras protein.

8. A chemotherapeutic composition comprising a compound of Claim 2.

9. The use of a compound as claimed in Claim 2 for the manufacture of a medicament for inhibiting farnesylation of Ras protein.

10. A compound of claim 1 which inhibits plasma membrane Ras protein localization and prevents transformation of normal cells into cancer cells, which compound comprises the sequence:

    S-(trans, trans)farnesyl-Cys-Aaa$^1$-Aaa$^2$-Xaa

    wherein
    Aaa$^1$ is an aliphatic amino acid;
    Aaa$^2$ is an aliphatic amino acid; and
    Xaa is an amino acid.

11. A compound of claim 10 selected from the group consisting of:
    S - (trans, trans) farnesyl-cys-Val-Leu-Ser;
    S - (trans, trans) farnesyl-cys-Val-Ile-Met; and
    S - (trans, trans) farnesyl-cys-Val-Val-Met.